# EUROPEAN PATENT SPECIFICATION

(11) **EP 1 588 671 B1**
(45) Date of publication and mention of the grant of the patent: **26.08.2009**
(21) Application number: 05076515.5
(22) Date of filing: 19.10.2001
(51) Int. Cl.: A61B 17/32, A61F 9/007

(54) **Method for determining a damping level of a handpiece/blade in an ultrasonic system**
Verfahren zur Bestimmung der Dämpfung eines Handstücks in einem Ultraschallschneidegerät
Méthode pour déterminer le degré d'amortissement d'un pièce à main/lame dans un système ultrasonique

(30) Priority: 20.10.2000 US 241888 P; 14.08.2001 US 930104
(43) Date of publication of application: 26.10.2005
(62) Divisional of application: 01308879.4
(73) Proprietor: ETHICON ENDO-SURGERY, INC., Cincinnati, Ohio 45242 (US)
(72) Inventor: Friedman, Allan L., Chicago, Illinois 60645-0000 (US); Donofrio, William T., Cincinnati, OH 45249 (US)
(74) Representative: Fisher, Adrian John

(56) References cited:
- EP-A- 0 359 217
- US-A- 6 117 152

## Description

### BACKGROUND OF THE INVENTION

### 1. FIELD OF THE INVENTION

The present invention generally relates to ultrasonic surgical systems and, more particularly, to a method for determining a damping level of a handpiece/blade in an ultrasonic system.

### 2. DESCRIPTION OF THE RELATED ART

It is known that electric scalpels and lasers can be used as a surgical instrument to perform the dual function of simultaneously effecting the incision and hemostatis of soft tissue by cauterizing tissues and blood vessels. However, such instruments employ very high temperatures to achieve coagulation, causing vaporization and fumes as well as splattering. Additionally, the use of such instruments often results in relatively wide zones of thermal tissue damage.

Cutting and cauterizing of tissue by means of surgical blades vibrated at high speeds by ultrasonic drive mechanisms is also well known. One of the problems associated with such ultrasonic cutting instruments is uncontrolled or undamped vibrations and the heat, as well as material fatigue resulting therefrom. In an operating room environment attempts have been made to control this heating problem by the inclusion of cooling systems with heat exchangers to cool the blade. In one known system, for example, the ultrasonic cutting and tissue fragmentation system requires a cooling system augmented with a water circulating jacket and means for irrigation and aspiration of the cutting site. Another known system requires the delivery of cryogenic fluids to the cutting blade.

It is known to limit the current delivered to the transducer as a means for limiting the heat generated therein. However, this could result in insufficient power to the blade at a time when it is needed for the most effective treatment of the patient. U.S. Patent No. 5,026,387 to *Thomas*, which is assigned to the assignee of the present application, discloses a system for controlling the heat in an ultrasonic surgical cutting and hemostasis system without the use of a coolant, by controlling the drive energy supplied to the blade. In the system according to this patent an ultrasonic generator is provided which produces an electrical signal of a particular voltage, current and frequency, e.g. 55,500 cycles per second. The generator is connected by a cable to a hand piece which contains piezoceramic elements forming an ultrasonic transducer. In response to a switch on the hand piece or a foot switch connected to the generator by another cable, the generator signal is applied to the transducer, which causes a longitudinal vibration of its elements. A structure connects the transducer to a surgical blade, which is thus vibrated at ultrasonic frequencies when the generator signal is applied to the transducer. The structure is designed to resonate at the selected frequency, thus amplifying the motion initiated by the transducer.

The signal provided to the transducer is controlled so as to provide power on demand to the transducer in response to the continuous or periodic sensing of the loading condition (tissue contact or withdrawal) of the blade. As a result, the device goes from a low power, idle state to a selectable high power, cutting state automatically depending on whether the scalpel is or is not in contact with tissue. A third, high power coagulation mode is manually selectable with automatic return to an idle power level when the blade is not in contact with tissue. Since the ultrasonic power is not continuously supplied to the blade, it generates less ambient heat, but imparts sufficient energy to the tissue for incisions and cauterization when necessary.

The control system in the *Thomas* patent is of the analog type. A phase lock loop (that includes a voltage controlled oscillator, a frequency divider, a power switch, a matching network and a phase detector), stabilizes the frequency applied to the hand piece. A microprocessor controls the amount of power by sampling the frequency, current and voltage applied to the hand piece, because these parameters change with load on the blade.

The power versus load curve in a generator in a typical ultrasonic surgical system, such as that described in the *Thomas* patent, has two segments. The first segment has a positive slope of increasing power as the load increases, which indicates constant current delivery. The second segment has a negative slope of decreasing power as the load increases, which indicates a constant or saturated output voltage. The regulated current for the first segment is fixed by the design of the electronic components and the second segment voltage is limited by the maximum output voltage of the design. This arrangement is inflexible since the power versus load characteristics of the output of such a system can not be optimized to various types of hand piece transducers and ultrasonic blades. The performance of traditional analog ultrasonic power systems for surgical instruments is affected by the component tolerances and their variability in the generator electronics due to changes in operating temperature. In particular, temperature changes can cause wide variations in key system parameters such as frequency lock range, drive signal level, and other system performance measures.

In order to operate an ultrasonic surgical system in an efficient manner, during startup the frequency of the signal supplied to the hand piece transducer is swept over a range to locate the resonance frequency. Once it is found, the generator phase lock loop locks on to the resonance frequency, continues to monitor the transducer current to voltage phase angle, and maintains the transducer resonating by driving it at the resonance frequency. A key function of such systems is to maintain the transducer resonating across load and temperature changes that vary the resonance frequency. However, these traditional ultrasonic drive systems have little to no flexibility with regards to adaptive frequency control. Such flexibility is key to the system's ability to discriminate undesired resonances. In particular, these systems can only search for resonance in one direction, i.e., with increasing or decreasing frequencies and their search pattern is fixed. The system cannot: (i) hop over other resonance modes or make any heuristic decisions, such as what resonance to skip or lock onto, and (ii) ensure delivery of power only when appropriate frequency lock is achieved.

The prior art ultrasonic generator systems also have little flexibility with regard to amplitude control, which would allow the system to employ adaptive control algorithms and decision making. For example, these fixed systems lack the ability to make heuristic decisions with regards to the output drive, e.g., current or frequency, based on the load on the blade and/or the current to voltage phase angle. It also limits the system's ability to set optimal transducer drive signal levels for consistent efficient performance, which would increase the useful life of the transducer and ensure safe operating conditions for the blade. Further, the lack of control over amplitude and frequency control reduces the system's ability to perform diagnostic tests on the transducer/blade system and to support troubleshooting in general.

Some limited diagnostic tests performed in the past involve sending a signal to the transducer to cause the blade to move and the system to be brought into resonance or some other vibration mode. The response of the blade is then determined by measuring the electrical signal supplied to the transducer when the system is in one of these modes. The ultrasonic system described in EP-A-1199047 possesses the ability to sweep the output drive frequency, monitor the frequency response of the ultrasonic transducer and blade, extract parameters from this response, and use these parameters for system diagnostics. This frequency sweep and response measurement mode is achieved via a digital code such that the output drive frequency can be stepped with high resolution, accuracy, and repeatability not existent in prior art ultrasonic systems.

A problem associated with the prior art ultrasonic systems is blade breakage or cracking at points of high stress on the blade. Breakage and cracking of blades are two major causes of the ultrasonic generator failing to acquire lock or failing to maintain longitudinal displacement. For example, as the crack develops both the frequency of oscillation and the magnitude of mechanical impedance change to such an extent that the ultrasonic generator can no longer locate the resonance of the hand piece/blade. A more advanced generator may be able to lock onto a transducer coupled to such a blade. However, a cracked blade has a reduced ability to oscillate in the longitudinal direction. In this situation, an increased ability to locate the desired resonance upon which to lock is not useful, and may actually mask the loss of optimal cutting conditions.

Further, burdened or gunked blades, i.e., blades with dried blood, skin, hair and desiccated tissue built up around the blade at the point where the sheath surrounds the blade, present a greater load than clean blades. In particular, the gunk results in a load on the blade, and represents an increase in the mechanical impedance of the transducer presented to the ultrasonic generator.

This phenomenon has the following unwanted consequence. Ultrasonic generators possess a maximum operating voltage beyond which optimal operation of the hand piece/blade is lost. Many ultrasonic drivers attempt to maintain a constant drive current level to the transducer to keep the displacement at the blade tip constant in the presence of varying loads on the blade. As the impedance of the transducer is increased (as a result of tissue pressure, gunked tissue, etc.), the drive voltage must be increased to maintain the drive current at a constant level. Eventually, the loading of the blade becomes great enough such that the voltage reaches a maximum level, and any further loading of the blade results in a reduction of the drive current signal level.

As the current level of the drive signal is reduced, the displacement will begin to fall. The generator can drive an increasing load only as long as the hand piece/blade is not loaded such that the resonance point becomes unrecognizable (due to degradation of the signal to noise ratio or an inability of the hand piece/blade to resonate). As a consequence, the tissue applied force at maximum power, the maximum tissue applied force before losing the resonance signal, and the cutting/coagulating ability of the blade between these two operating points, become degraded.

In addition to the problems associated with loads on the blade, there is a buildup of heat at the coagulum. This buildup absorbs energy from the blade, and heats both the blade and sheath at this location. A cracked or broken blade loses the ability to resonant as well as a blade which is in good condition, and thus should be discarded. However, a gunked blade can be cleaned or used, and resonates as well as a new blade. In an operating room, access to either cracked and gunked blades for visual inspection is not practical. However, it is advantageous to differentiate between broken blades and those which are gunked, but otherwise in good condition, because a user can quickly and with confidence decide whether to discard or to clean an expensive blade. Cleaning a blade which is gunked verses discarding what is otherwise a good blade results in a substantial reduction in purchasing costs which are passed on to hospital patients as a savings.

Detection of debris on the blade, and the determination of the condition of tissue that the blade is in contact with are additional problems associated with conventional ultrasonic systems. Some ultrasonic blades are equipped with a sheath which covers the blade. The majority of the sheath is not in contact with the blade. Space (voids) between the sheath and the blade permits the blade to move freely. During use, this space can become filled with debris such as blood and tissue. This debris has a tendency to fill the space between the sheath and blade, and increase mechanical coupling between the blade and the sheath. As a result, undesired loading of the blade may increase, the temperature of the blade sheath may increase and the energy delivered to the tip may be reduced. In addition, if the debris sufficiently coagulates/hardens inside the sheath, the ability of the generator to initiate blade vibration while in contact with tissue may be prohibited. Moreover, vibration/start up of the blade in free air may also be inhibited.

EP0359217 discloses a driver system for an ultrasonic probe for allowing a user to have proportional control of the power dissipated in the probe in accordance with the position of power dissipation controls operable by the user and for automatically tuning upon user request such that the driving frequency is equal to the mechanical resonant frequency of said probe and such that the reactive component of the load impedance represented by said probe is tuned out.

US 6,117,152 discloses a multi-function ultrasonic surgical instrument operable to perform a number of surgical procedures including: 1) scoring of tissue to a controlled depth, 2) coaptive transection of vessels utilizing a clamping mechanism, 3) coagulation under tension with a blunt edge, 4) coaptive coagulation and ablation, and 5) tissue nibbling.

### SUMMARY OF THE INVENTION

The present invention provides a method for : determining a damping level of a hand piece/blade in an ultrasonic system as claimed in claim 1, determining a relative dampening level of a blade in an ultrasonic system as claimed in claim 4 and determining relative level of dampening of a hand piece/blade in an ultrasonic system as claimed in claim 8.

In another embodiment of the invention, the condition and effect of debris upon the sheath is used to detect debris inside the sheath. The debris dampens the blade vibrations, and also reduces the Q of the hand piece/blade system. Thus, debris is detected by measuring the extent of blade dampening or the reduction of the Q of the hand piece/blade. This effect is pronounced while the blade is held "in the air," since the variable causes of dampening are mostly related to debris. In particular, contact with tissue will load or dampen the blade. If the blade is held in air so it does not touch the tissue, only the gunk will load the blade. This measurement can be obtained when initiated/directed by the user and/or automatically when the impedance of the hand piece/blade is distinctly low, thus indicating that the blade is not in contact with tissue.

In a further embodiment of the invention, real time detection of debris on the sheath (i.e., a dampening test) is performed. This is achieved by measuring the impedance of the hand piece/blade and determining when the blade is not in contact with a working surface (i.e., skin tissue). The damping test is performed whenever a measurement indicates "no contact." With Blade Identification (Blade ID) in use, each type of blade will possess a specific assigned dampening or Q level. Blade ID is the use of a code stored in non-volatile memory located in the hand piece to identify whether a blade is connected to the hand piece or to identify the type of blade connected to the hand piece.

If the console driving the blade detects a shift in dampening or Q, upon comparison of the dampening value or Q to an expected value stored in the hand piece, such a shift can indicate the degree of influence that the debris is exerting upon the hand piece/blade. This result is advantageously useful if, during periodic impedance monitoring, removal of the blade is not detected, even though the dampening has substantially changed. For example, if the blade is not used for an extended period of time, blood/tissue which enters the sheath gap during use will coagulate and substantially dampen the blade. This change in dampening can be observed and detected, and the user can be alerted to the change.

In an additional embodiment of the invention, the condition of tissue is determined. A blade in contact with tissue possesses a dampened response which is relative to the condition of the tissue and the pressure applied. For a given blade, tissue condition and contact pressure, the amount of dampening changes as the tissue condition changes. Consequently, the condition of tissue is determined by obtaining relative measurements of dampening while the blade is in contact with the tissue. This is accomplished by periodically interrupting the normal drive signal to the transducer, providing a test drive signal to obtain a brief dampening measurement, and then reapplying the normal drive signal to the transducer. This does not degrade the overall performance of the ultrasonic system, and does not interrupt the continuous use of the system.

The method can be advantageously used to focus on a single event, such as the coagulation of a vessel. When the user of the ultrasonic system begins to coagulate the blood vessel, the console measures the initial dampening level and periodically continues dampening measurements until the dampening level has adequately changed and/or the rate of dampening has appropriately changed. When the appropriate dampening response is reached (e.g., when tissue of one type or condition has been severed and the blade has encountered tissue of another type or condition), the console indicates the status to the user or stops/reduces energy delivery to the hand piece/blade. The energy delivery is adjustable in real-time according to the measured on-going dampening levels. The dampening level is displayable for consideration by the user, or is usable in an algorithm to control energy delivery to the hand piece/blade.

It is also desirable to know the relative condition of skin tissue, especially the condition of the tissue which has been altered by ultrasonic energy. Assessing the condition of tissue permits the proper adjustment of the energy applied to the tissue, and also permits the indication of when adequate cauterization, dessication, or other tissue effects have occurred. Together, these provide a means to determine whether additional energy or whether an extension of the application time of the energy is required. Further, the assessment of the tissue condition permits the avoidance of insufficient energy applications and insufficient tissue effects (i.e., poor tissue coagulation or poor tissue cauterization), which prevent application of excessive amounts of ultrasonic energy to the skin tissue which can harm surrounding tissue in the area of blade usage.

### BRIEF DESCRIPTION OF THE DRAWINGS

The foregoing and other advantages and features of the invention will become more apparent from the detailed description of the preferred embodiments of the invention given below with reference to the accompanying drawings in which:
FIG. 1 is an illustration of impedance vs frequency plots for an ultrasonic blade which is cracked, gunked or good when driven at a low signal level or a high signal level;
FIG. 2 is an illustration of phase vs frequency plots for an ultrasonic blade which is cracked, gunked or good when driven at a low signal level or a high signal level;
FIG. 3 is an illustration of impedance vs frequency plots for an ultrasonic blade which is cracked or has completely broken away from a hand piece when driven at a low signal level or a high signal level;
FIG. 4 is an illustration of a console for an ultrasonic surgical cutting and hemostasis system, as well as a hand piece and foot switch;
FIG. 5 is a schematic view of a cross section through the ultrasonic scalpel hand piece of the system of FIG. 4;
FIGS. 6(a) and 6(b) are block diagrams illustrating an ultrasonic generator;
FIGS. 7(a) and (b) are flow charts; ;
FIGS. 8(a) and 8(b) are flow charts ;
FIG. 9 is a flow chart illustrating an embodiment of the invention;
FIG. 10 is a flow chart illustrating a further embodiment of the invention; and
FIG. 11 is a flow chart illustrating an additional embodiment of the invention.

### DETAILED DESCRIPTION OF THE PREFERRED EMBODIMENTS

Impedance measurements of mechanical or acoustic systems obtained at high excitation levels provides much more information than impedance measurements obtained at low excitation levels. Moreover, comparisons of impedance measurements between low and high energy excitation levels provide even more detailed information about the condition of the hand piece/blade. The condition of the hand piece/blade falls into three main categories.

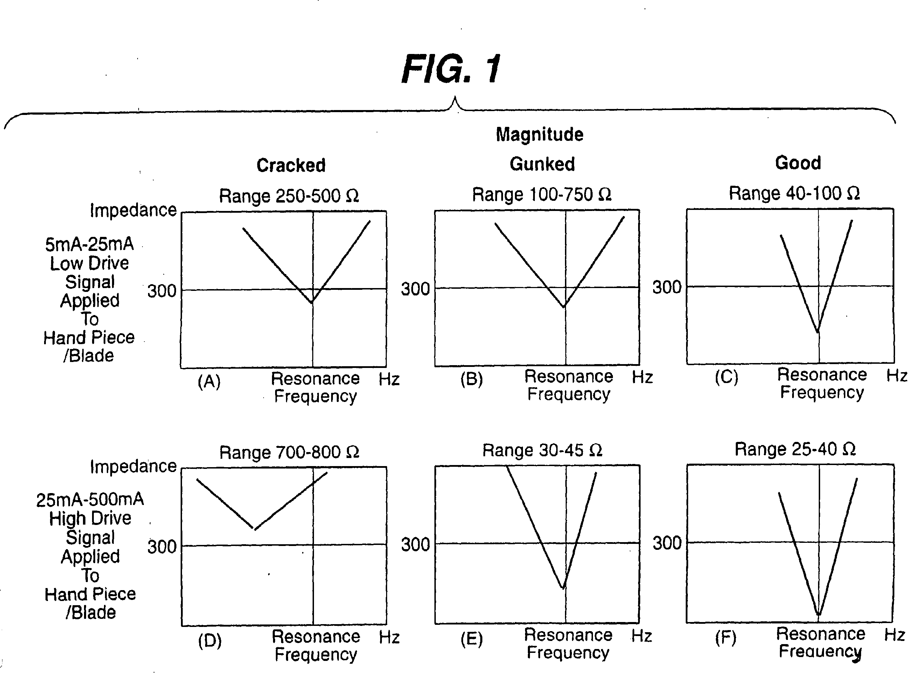

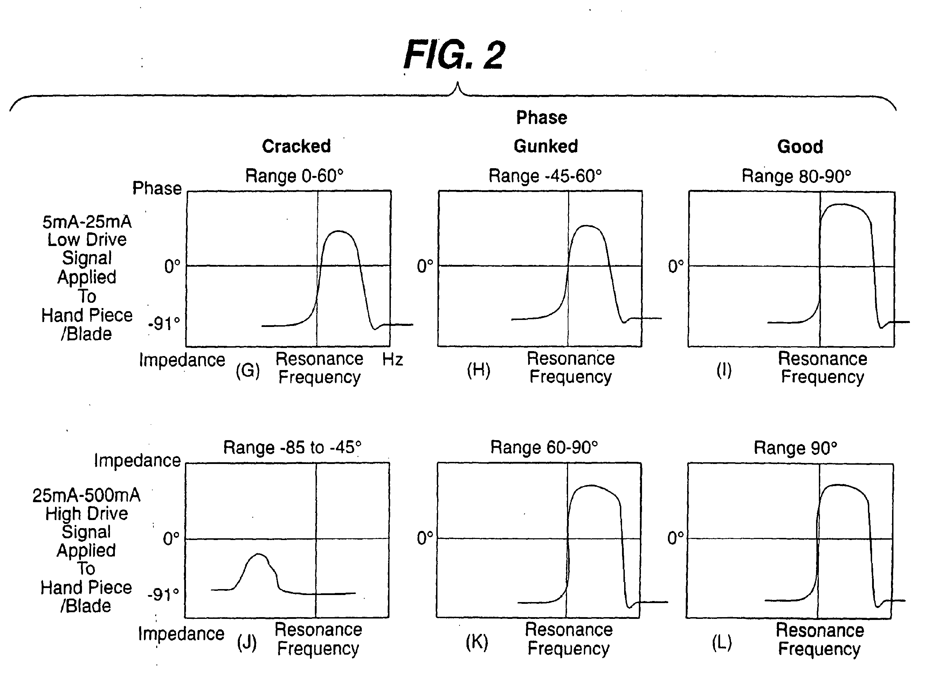

Firstly, gunked blades and new clean blades belong to the same category because silicon anti-node supporters and other mechanical inefficiencies, such as mechanical resistance in the longitudinal direction of the blade, have the same dampening effect as gunk upon the hand piece/blade. In particular, clean/gunked systems become much better resonators as the excitation amplitude is increased, that is they become higher Q systems (the minimum impedance gets markedly lower and the maximum phases get markedly higher; see FIG. 1 and compare the impedance vs. frequency plot shown in B to the impedance vs. frequency plot shown in E, and see FIG. 2 and compare the phase vs. frequency plot shown in H to the phase vs. frequency plot shown in K). The degree of improvement is relative to the loading effect of the gunk involved. As the excitation level changes, there is a minimal change in the resonance frequency which is close to the resonance frequency of a clean hand piece/blade. At a low excitation level, such as 5mA, a cracking or slightly cracked blade is generally self healing and looks very much like a gunked blade (see FIG. 1 and compare the impedance vs. frequency plot shown in A to the impedance *vs*. frequency plot shown in B, and see FIG. 2 and compare the phase vs. frequency plot shown in G to the phase vs. frequency plot shown in H). The self healing characteristic, in which at a molecular level the blade becomes more homogeneous if not overly excited, results in an optimally tuned system. At low excitation levels, the surfaces at the interface of the crack do not behave like disjoint surfaces, and are held in close contact to each other by the parts of the blades which are still intact. In this situation, the system appears "healthy."

Secondly, at larger excitation levels, such as 25mA or greater, stresses at the crack become large enough such that the portion of the blade which is distal to the crack no longer acts as if it is intimately connected to the proximal portion of the blade. A characteristic of these hand piece/blades is their non-linear behavior (i.e., very sharp non-continuous changes in impedance magnitudes and phase) which occur as the resonance frequency is approached and the stresses in the shaft of the hand piece become large. As the frequency approaches resonance of the "intact blade", the stresses become increasingly greater until at a certain point the blade suddenly becomes disjointed at the crack. This effectively shortens the blade, and the resonator or blade will possess completely different resonance impedance characteristics. Typically, the impedance of such a shorter blade results in a hand piece/blade which possesses a lower Q, as well as a lower frequency of resonance (see FIG. 1 and compare the respective impedance vs. frequency plots shown in A and C to the respective impedance *vs*. frequency plots shown in D and F, and see FIG. 2 and compare the respective phase vs. frequency plots shown in G and I to the respective phase vs. frequency plots shown in J and L).

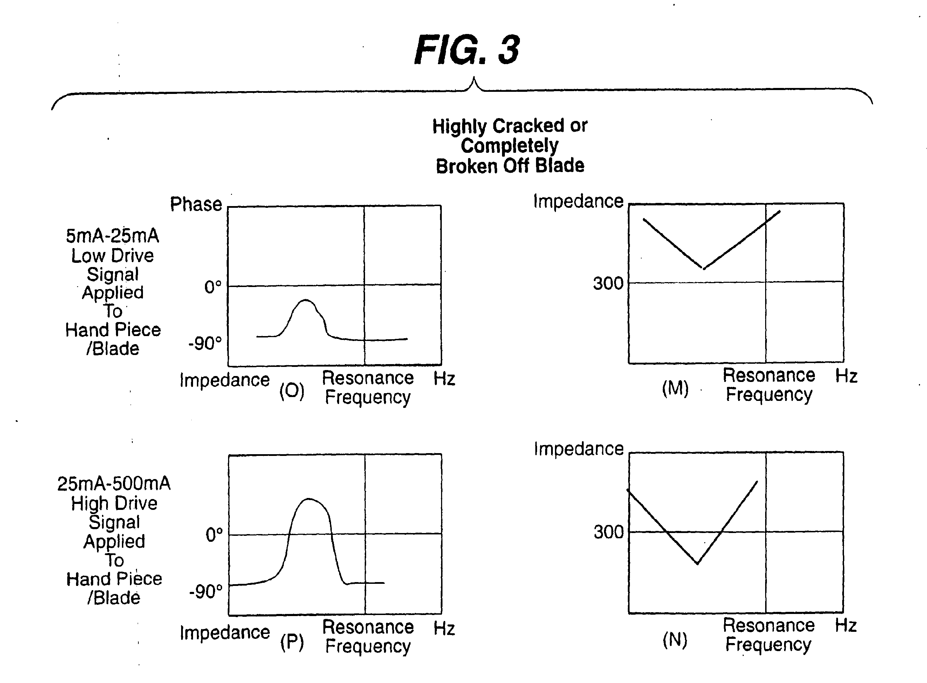

Lastly, severely cracked blades include, but are not limited to, blades having tips which have completely fallen off due to mechanical stress acting on the blades. These blades are substantially equivalent to gunked blades. However, they are not useful for cutting/coagulating tissue in longitudinal directions. Such blades appear to behave similarly in that they present improved (if only marginally) impedance characteristics at higher excitation levels, and their frequency of resonance is not affected by higher excitation levels. However, they can be differentiated from gunked blades due to their extremely high impedance level. This requires absolute measurements, but only coarse levels of precision are required. Generally, the resonance frequency of the transducer or blade is shifted far away from the normal resonance that is typically used for a specific ultrasonic system. This shift is usually a downward shift of the resonance frequency of about 2 kilohertz. When excited with a higher level of current and compared with a lower level of current, the impedance magnitude, resonance frequency and maximum phase at resonance are quantitatively far different than the corresponding characteristics of blades which are only gunked (see FIG. 3 and compare the impedance vs. frequency plot shown in M to the impedance *vs*. frequency plot shown in N, and compare the phase vs. frequency plot shown in O to the phase vs. frequency plot shown in P). In this case, the hand piece/blade typically possesses a magnitude of impedance at resonance which is approximately 400 ohms higher for cracked blades than that of heavily gunked but otherwise good blades. Of note, Figs. 1-3 show values that are exemplify a particular US system, and absolute values are dependent upon actual the actual design of the system.

Most broken or cracked blades have self healing characteristics associated with them. The self healing characteristic, in which at a molecular level the blade becomes more homogeneous if not overly excited, results in an optimally tuned system. This homogeneity is disturbed at a high excitation level, resulting in an untuned system. When cracked or broken blades are un-energized for an extended period of time, or if energized at a low intensity for a period of time, such blades present a mechanical impedance to the ultrasonic generator that is closer to the mechanical impedance which is exhibited by an unbroken blade. At high excitation levels, the portion of the blade distal to the crack is no longer intimately connected to the hand piece/blade. The effect of the high excitation level upon the blade is that the portion of the blade proximal to the crack "bangs" against the portion of the blade distal to the crack, which causes a loading effect which is greater than the loading effect at low excitation displacement levels.

In other words, in the frequency range of approximately 1,000 Hz, centered around the resonance frequency of an unbroken blade, the same type of broken blade will exhibit one impedance sweep characteristic at a low voltage excitation of the drive transducer and another at a high voltage excitation level. In contrast, an unbroken blade exhibits the same impedance at both excitation levels, as long as the impedance measurement is performed quickly enough, or at a low enough displacement level such that the transducer or the blade does not overheat. Heat causes the resonance point to shift downwards in frequency. This heating effect is most prevalent when the magnitude of the excitation frequency approaches the resonance frequency due to gunk.

In addition, an excitation threshold exists, below which the blade "self heals" and presents increasingly "tuned" impedance levels (overtime) to the driving elements, and above which the crack presents a discontinuity to the homogeneity of the blade. Thus, below this threshold, the impedance characteristic may exhibit the same characteristic for all excitation levels. The blade may also appear to be healing itself at these lowered excitation levels. Above this excitation threshold, the impedance may possess a different appearance than the low impedance measurements, but may still not change with increasing levels of excitation. This excitation threshold is different for each type of blade as well as each cracked location on the blade, and is modulated by the amount of gunk loading the distal part of the blade.

Some of the impedance differences seen in a system containing a broken blade (which are not seen in a system containing an unbroken blade), when first driven with a low excitation current and then with a high excitation current, are a lower Q (i.e., a lower minimum impedance) over a frequency span centered about the resonance frequency of an unbroken blade, i.e., a higher minimum impedance and/or a lower maximum impedance. It could also mean a higher "phase margin", i.e., Fa-Fr (where Fa-Fr is anti-resonance frequency minus the resonance frequency, respectively). Other differences are a higher impedance at a frequency slightly above the anti-resonance frequency of the normally operating system, a higher impedance at a frequency slightly below the resonance point of a properly working system, or a large change in the resonance frequency. Gunked or loaded blades connected to a drive system exhibit somewhat opposite effects to that of a cracked blade. A system loaded in this manner exhibits an increasingly improved Q around the resonance point as the excitation voltage is increased.

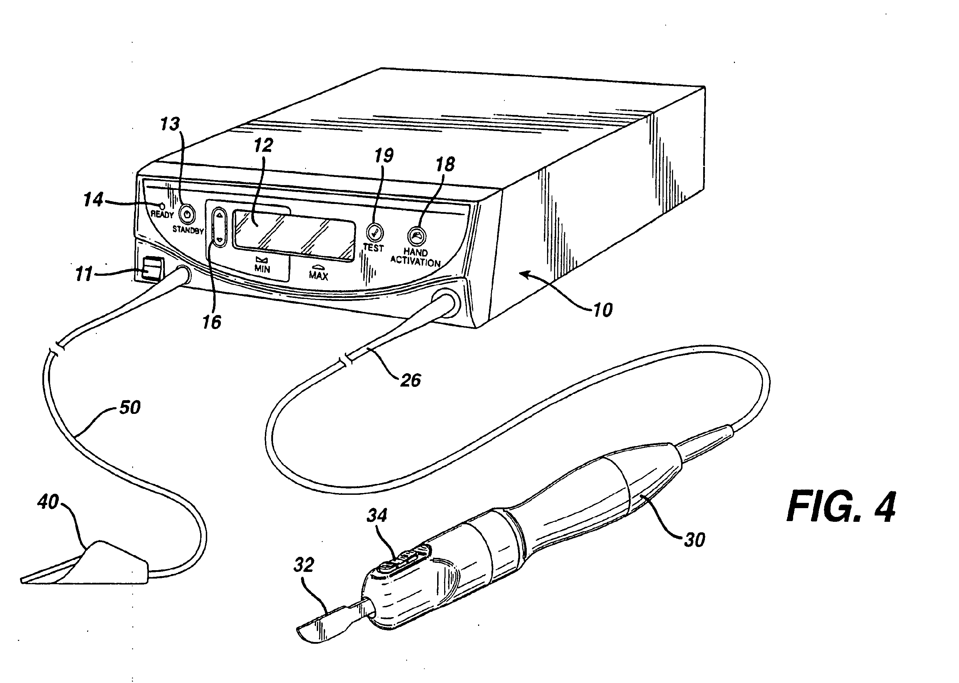

FIG. 4 is an illustration of a system. By means of a first set of wires in cable 20, electrical energy, i.e., drive current, is sent from the console 10 to a hand piece 30 where it imparts ultrasonic longitudinal movement to a surgical device, such as a sharp scalpel blade 32. This blade can be used for simultaneous dissection and cauterization of tissue. The supply of ultrasonic current to the hand piece 30 may be under the control of a switch 34 located on the hand piece, which is connected to the generator in console 10 via wires in cable 20. The generator may also be controlled by a foot switch 40, which is connected to the console 10 by another cable 50. Thus, in use a surgeon may apply an ultrasonic electrical signal to the hand piece, causing the blade to vibrate longitudinally at an ultrasonic frequency, by operating the switch 34 on the hand piece with his finger, or by operating the foot switch 40 with his foot.

The generator console 10 includes a liquid crystal display device 12, which can be used for indicating the selected cutting power level in various means such, as percentage of maximum cutting power or numerical power levels associated with cutting power. The liquid crystal display device 12 can also be utilized to display other parameters of the system. Power switch 11 is used to turn on the unit. While it is warming up, the "standby" light 13 is illuminated. When it is ready for operation, the "ready" indicator 14 is illuminated and the standby light goes out. If the unit is to supply maximum power, the MAX button 15 is depressed. If a lesser power is desired, the MIN button 17 is activated. The level of power when MIN is active is set by button 16.

When power is applied to the ultrasonic hand piece by operation of either switch 34 or 40, the assembly will cause the surgical scalpel or blade to vibrate longitudinally at approximately 55.5 kHz, and the amount of longitudinal movement will vary proportionately with the amount of driving power (current) applied, as adjustably selected by the user. When relatively high cutting power is applied, the blade is designed to move longitudinally in the range of about 40 to 100 microns at the ultrasonic vibrational rate. Such ultrasonic vibration of the blade will generate heat as the blade contacts tissue, i.e., the acceleration of the blade through the tissue converts the mechanical energy of the moving blade to thermal energy in a very narrow and localized area. This localized heat creates a narrow zone of coagulation, which will reduce or eliminate bleeding in small vessels, such as those less than one millimeter in diameter. The cutting efficiency of the blade, as well as the degree of hemostasis, will vary with the level of driving power applied, the cutting rate of the surgeon, the nature of the tissue type and the vascularity of the tissue.

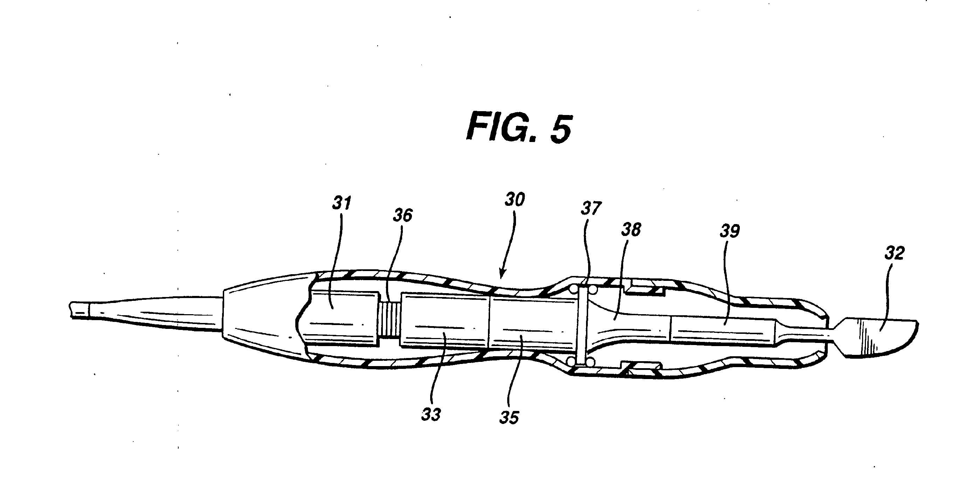

As illustrated in more detail in FIG. 5, the ultrasonic hand piece 30 houses a piezoelectric transducer 36 for converting electrical energy to mechanical energy that results in longitudinal vibrational motion of the ends of the transducer. The transducer 36 is in the form of a stack of ceramic piezoelectric elements with a motion null point located at some point along the stack. The transducer stack is mounted between two cylinders 31 and 33. In addition a cylinder 3 5 is attached to cylinder 33, which in turn is mounted to the housing at another motion null point 37. A horn 38 is also attached to the null point on one side and to a coupler 39 on the other side. Blade 32 is fixed to the coupler 39. As a result, the blade 32 will vibrate in the longitudinal direction at an ultrasonic frequency rate with the transducer 36. The ends of the transducer achieve maximum motion with a portion of the stack constituting a motionless node, when the transducer is driven with a current of about 380mA RMS at the transducers' resonant frequency. However, the current providing the maximum motion will vary with each hand piece and is a valve stored in the non-volatile memory of the hand piece so the system can use it.

The parts of the hand piece are designed such that the combination will oscillate at the same resonant frequency. In particular, the elements are tuned such that the resulting length of each such element is one-half wavelength. Longitudinal back and forth motion is amplified as the diameter closer to the blade 32 of the acoustical mounting horn 38 decreases. Thus, the horn 38 as well as the blade/coupler are shaped and dimensioned so as to amplify blade motion and provide harmonic vibration in resonance with the rest of the acoustic system, which produces the maximum back and forth motion of the end of the acoustical mounting horn 38 close to the blade 32. A motion at the transducer stack is amplified by the horn 38 into a movement of about 20 to 25 microns. A motion at the coupler 39 is amplified by the blade 32 into a blade movement of about 40 to 100 microns.

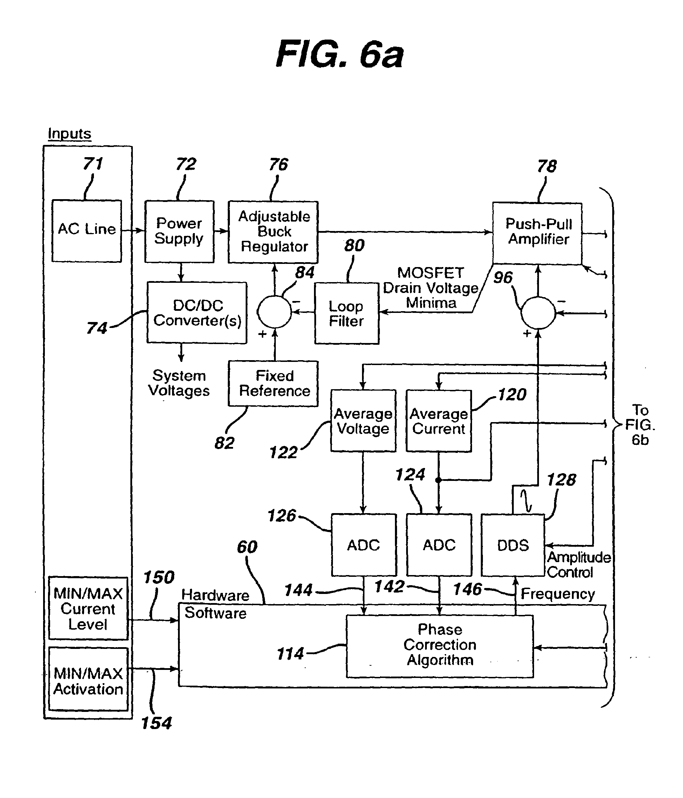

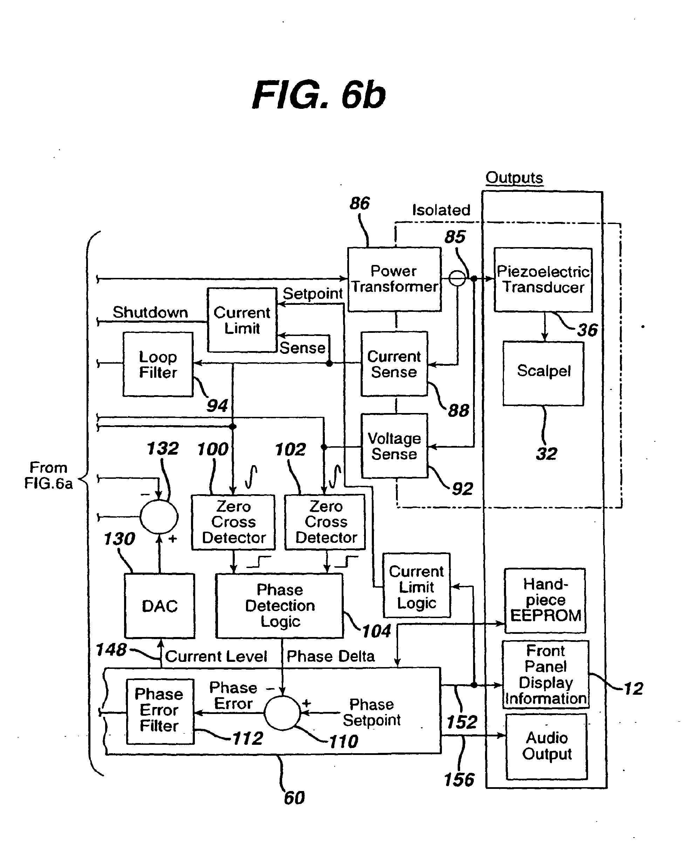

The system which creates the ultrasonic electrical signal for driving the transducer in the hand piece is illustrated in FIGS. 6(a) and 6(b). This drive system is flexible and can create a drive signal at a desired frequency and power level setting. A DSP 60 or microprocessor in the system is used for monitoring the appropriate power parameters and vibratory frequency as well as causing the appropriate power level to be provided in either the cutting or coagulation operating modes. The DSP 60 or microprocessor also stores computer programs which are used to perform diagnostic tests on components of the system, such as the hand piece/blade.

For example, under the control of a program stored in the DSP or microprocessor 60, such as a phase correction algorithm, the frequency during startup can be set to a particular value, e.g., 50 kHz. It can than be caused to sweep up at a particular rate until a change in impedance, indicating the approach to resonance, is detected. Then the sweep rate can be reduced so that the system does not overshoot the resonance frequency, e.g., 55 kHz. The sweep rate can be achieved by having the frequency change in increments, e.g., 50 cycles. If a slower rate is desired, the program can decrease the increment, e.g., to 25 cycles which both can be based adaptively on the measured transducer impedance magnitude and phase. Of course, a faster rate can be achieved by increasing the size of the increment. Further, the rate of sweep can be changed by changing the rate at which the frequency increment is updated.

If it is known that there is a undesired resonant mode, e.g., at say 51 kHz, the program can cause the frequency to sweep down, e.g., from 60 kHz, to find resonance. Also, the system can sweep up from 50 kHz and hop over 51 kHz where the undesired resonance is located. In any event, the system has a great degree of flexibility

In operation, the user sets a particular power level to be used with the surgical instrument. This is done with power level selection switch 16 on the front panel of the console. The switch generates signals 150 that are applied to the DSP 60. The DSP 60 then displays the selected power level by sending a signal on line 152 (FIG. 6(b)) to the console front panel display 12. Further, the DSP or microprocessor 60 generates a digital current level signal 148 that is converted to an analog signal by digital-to-analog converter (DAC) 130.

To actually cause the surgical blade to vibrate, the user activates the foot switch 40 or the hand piece switch 34. This activation puts a signal on line 154 in FIGS. 6(a). This signal is effective to cause power to be delivered from push-pull amplifier 78 to the transducer 36. When the DSP or microprocessor 60 has achieved lock on the hand piece transducer resonance frequency and power has been successfully applied to the hand piece transducer, an audio drive signal is put on line 156. This causes an audio indication in the system to sound, which communicates to the user that power is being delivered to the hand piece and that the scalpel is active and operational.

In order to obtain the impedance measurements and phase measurements, the DSP 60 and the other circuit elements of FIGS. 6(a) and 6(b) are used. In particular, push-pull amplifier 78 delivers the ultrasonic signal to a power transformer 86, which in turn delivers the signal over a line 85 in cable 26 to the piezoelectric transducers 36 in the hand piece. The current in line 85 and the voltage on that line are detected by current sense circuit 88 and voltage sense circuit 92. The current and voltage sense signals are sent to average voltage circuit 122 and average current circuit 120, respectively, which take the average values of these signals. The average voltage is converted by analog-to-digital converter (ADC) 126 into a digital code that is input to DSP 60. Likewise, the current average signal is converted by analog-to-digital converter (ADC) 124 into a digital code that is input to DSP 60. In the DSP the ratio of voltage to current is calculated on an ongoing basis to give the present impedance values as the frequency is changed. A significant change in impedance occurs as resonance is approached.

The signals from current sense 88 and voltage sense 92 are also applied to respective zero crossing detectors 100, 102. These produce a pulse whenever the respective signals cross zero. The pulse from detector 100 is applied to phase detection logic 104, which can include a counter that is started by that signal. The pulse from detector 102 is likewise applied to logic circuit 104 and can be used to stop the counter. As a result, the count which is reached by the counter is a digital code on line 104, which represents the difference in phase between the current and voltage. The size of this phase difference is also an indication of resonance. These signals can be used as part of a phase lock loop that cause the generator frequency to lock onto resonance, e.g., by comparing the phase delta to a phase set point in the DSP in order to generate a frequency signal to a direct digital synthesis (DDS) circuit 128 that drives the push-pull amplifier 78.

Further, the impedance and phase values can be used as indicated above in a diagnosis phase of operation to detect if the blade is loose. In such a case the DSP does not seek to establish phase lock at resonance, but rather drives the hand piece at particular frequencies and measures the impedance and phase to determine if the blade is tight.

Since the DSP has measured and stored values of impedance and phase at particular frequencies and excitation levels, it can plot responses such as those in FIGS. 1-3. Thus, it can calculate the Q of the hand piece as well.

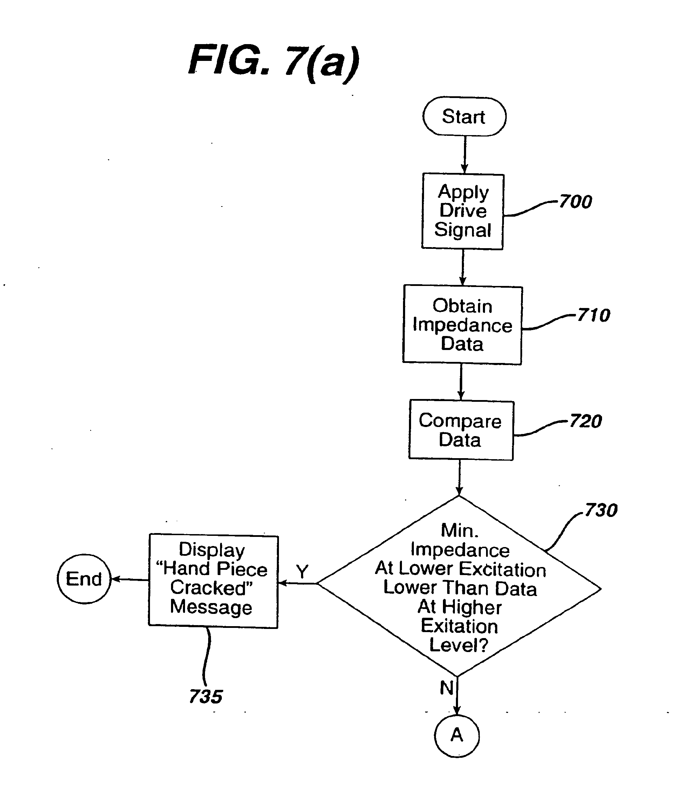

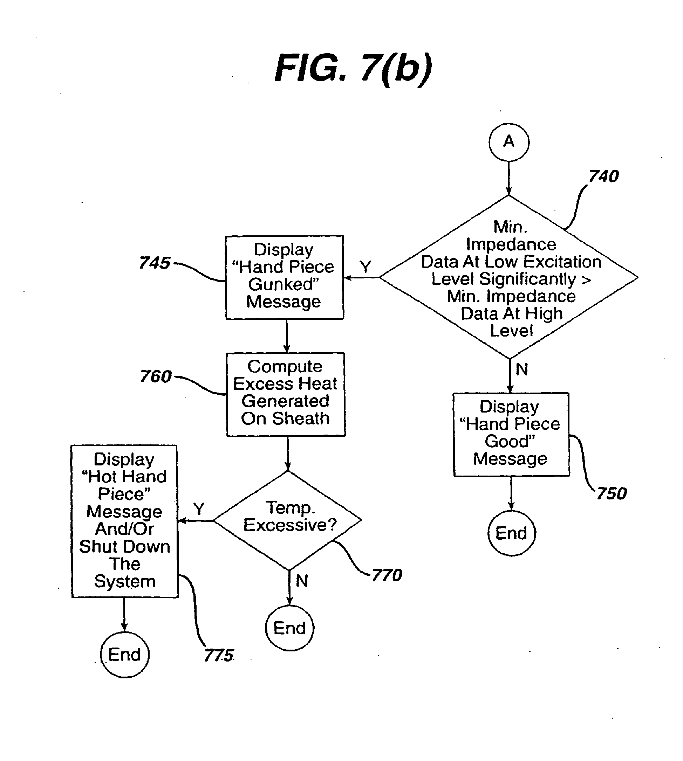

FIGS. 7(a) and 7(b) are flow charts. Under control of the program stored in the DSP or microprocessor 60 shown in FIGS. 6(a) and 6(b), the method is implemented by using the ultrasonic driver unit to excite the hand piece/blade and obtain impedance data over a frequency range of 50 to 60 kilohertz, as indicated in step **700**. Magnitude of impedance and phase of impedance data is obtained for two or more excitation levels ranging from a first current level to second current level, such as from 5mA to 50mA, as indicated in step **710.** Data within this range is collected in any order, including sweeping up or down in a discontinuous sampling sequence. To identify or discriminate between gunked and cracked blades, comparisons are performed between characteristics measurements, such as the magnitude of the lowest impedance obtained, the maximum phase between the current and the voltage, the resonance frequency of the blade, and/or an evaluation of the non-linearity and/or continuousness of the measured data, as indicated in step 720.

If the impedance data sweep(s) at a lower excitation level reveal that the minimum impedance magnitude is lower than the minimum impedance magnitude obtained at a higher excitation level (step 730), then the blade or the hand piece is cracked, and a "Blade Cracked" message is displayed on the LCD 12, as indicated in step 735. Alternatively, whether the difference between the frequency of resonance at a high level and the frequency of resonance at a low level is less than or equal to a threshold, such as 20 Hz, can be used to indicated whether a cracked blade exists. If, on the other hand, the lower excitation sweep(s) show little or no change in resonance frequency or a higher minimum impedance than the higher excitation sweeps (step **740),** then the blade or hand piece is gunked, and a "Gunked Blade" message is displayed on the LCD 12, as indicated in step **745.** Further, the amount of gunking is determined by the differences in the impedance magnitudes which are obtained, and communicated to the user during display of the "Blade Gunked" message. The amount of excess heat generation on the sheath at the location of the gunk is computed, as indicated in step 760. Excess heat may be estimated by calculating the relative difference in magnitude of the impedance measurements. If the temperature build up of heat will be excessive, a "Hot Blade" warning message is displayed on the LCD 12 and/or the user is instructed to shut down the system, as indicated in step **775.** If, on the other hand, the heat will not be excessive, the diagnostic test is terminated. Of note, the hot blade warning message is dependant on the blade characteristics. Heat generated within a particular blade design may be determined by using an I²R power-to-heat conversion for a given blade. It should be noted that the all of described measurements procedures may be performed using the DSP or microprocessor 60 in the ultrasonic generator. However, other devices may also be used to perform the measurements, such as a CPU, a Programmable Logic Device (PLD), or the like.

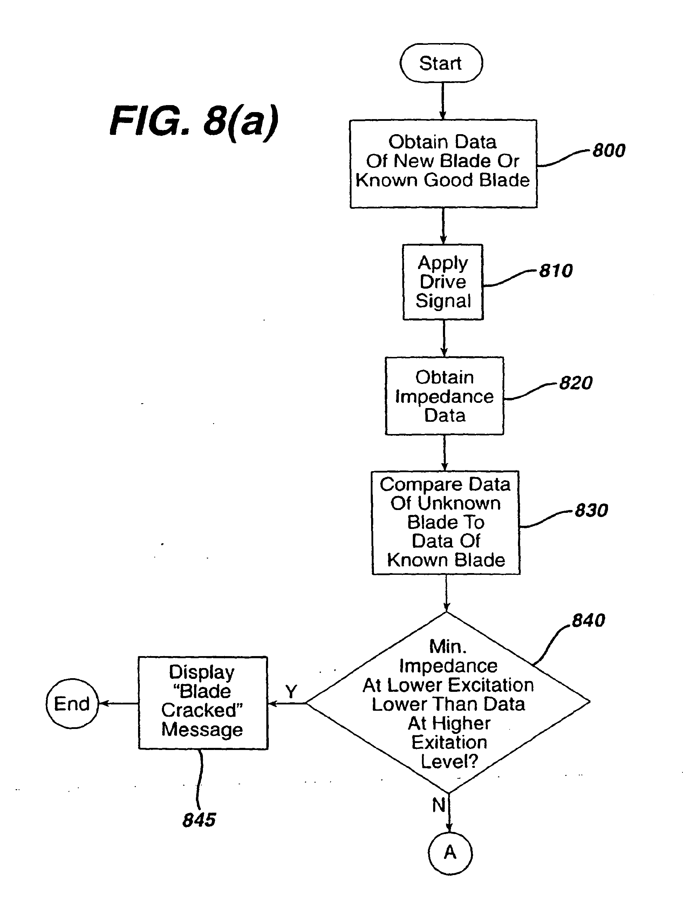

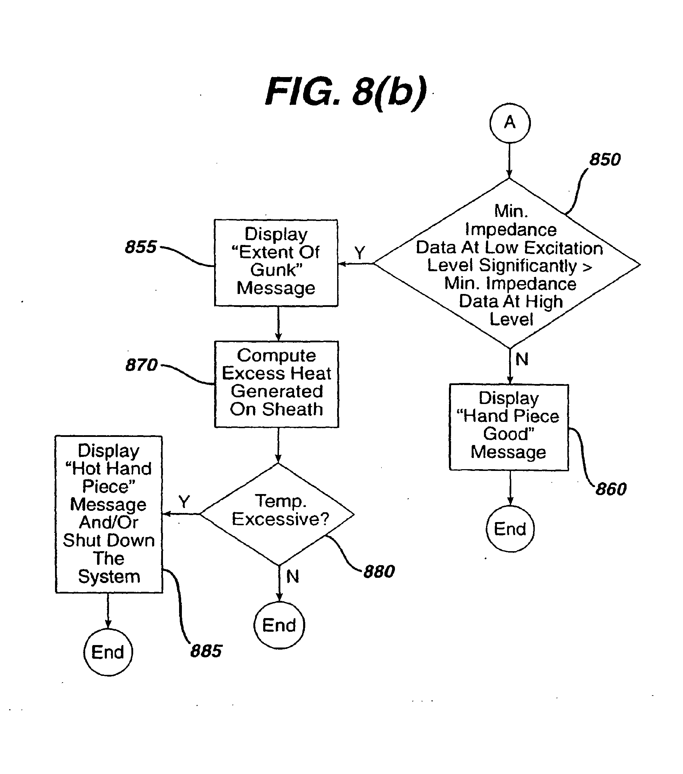

FIGS. 8(a) and 8(b) are flow charts To increase the accuracy of the measurements, measurements of data from an initial test of a known good blade is compared to measurement data of a blade in an unknown condition. A threshold based on defined boundaries or ratios to a known good blade characteristics is calculated. As a result, testing accuracy is increased and less pronounced mal-effects on blades are detected. In addition, the ability to distinctly determine the extent of gunking is also provided. This is due to the attainment and use of a greater level of blade-specific measurement data for comparison, rather than the use of expected behavior data associated with generic good blades.

In an embodiment, instead of obtaining data by performing a test of the actual blade on the hand piece, the data can be obtained from a data source for the particular blade model which is in the blade ID or entered in the console, or the like. For details relating to blade ID, reference is made to EP-A-1260185.

The method permits the determination of whether the blade is in a severe condition or whether it is marginally problematic. In this case, the user can try to clean the blade and perform another test to measure the progress of cleaning and to help the user determine whether the cleaning of the blade is effective or ineffective. In embodiments, the "grading" may be used without the benefit of "known good blade" characteristics by providing a relative gunk score before and after cleaning to indicate how effectively the blade was cleaned. In alternative embodiments, the method is periodically initiated automatically by the console of the generator.

Under control of the program stored in the DS P or microprocessor 60 shown in FIGS. 6(a) and 6(b), the method is implemented by obtaining impedance data of a new blade or blade which is in good condition, as indicated in step **800.** The ultrasonic driver unit is used to excite the hand piece/blade and obtain impedance data over a frequency range of 50 to 60 kilohertz, as indicated in step **810**. Magnitude of impedance and phase of impedance data is obtained for two or more excitation levels ranging from a first current level to second current level, such as from 5mA to 50mA, as indicated in step **820.** Data within this range is collected in any order, including sweeping up or down in a discontinuous sampling sequence. To identify or discriminate between gunked and cracked blades, comparisons are performed between characteristics measurements, such as the magnitude of the lowest impedance obtained, the maximum phase between the drive current and the drive voltage, the resonance frequency of the blade, and/or an evaluation of the non-linearity and/or continuousness of the measured data, as indicated in step **830**.

If the impedance data sweep(s) at a lower excitation level reveal that the minimum impedance magnitude is lower than the minimum impedance magnitude obtained at a higher excitation level (step **840**), then the blade or the hand piece is cracked, and a "Blade Cracked" message is displayed on the LCD 12, as indicated in step **845.** Alternatively, whether the difference between the frequency of resonance at a high level and the frequency of resonance at a low level is less than or equal to a threshold, such as 20 Hz, can be used to indicated whether a cracked blade exists. If, on the other hand, the lower excitation sweep(s) show little or no change in resonance frequency or a higher minimum impedance than the higher excitation sweeps (step 850), then the blade or hand piece is gunked, and a "Extent of Gunk" message is displayed on the LCD 12, as indicated in step **855.** Further, the amount of gunking is determined by the differences in the impedance magnitudes which are obtained, and communicated to the user during display of the "Extent of Gunk" message. The amount of excess heat generation on the sheath at the location of the gunk is computed, as indicated in step **870.** Excess heat may be estimated by calculating the relative difference in magnitude of the impedance measurements. If the temperature build up of heat will be excessive, a "Hot Blade" warning message is displayed on the LCD 12 and/or the user is instructed to shut down the system, as indicated in step **885.** If, on the other hand, the heat will not be excessive, the diagnostic test is terminated. As stated previously, the hot blade warning message is dependant on the blade characteristics. Heat generated within a particular blade design may be determined by using an I²R power-to-heat conversion for a given blade. In addition, the described measurement procedures may also be performed using the DSP or microprocessor 60 in the ultrasonic generator. However, other devices may also be used to perform the measurements, such as a CPU, a Programmable Logic Device (PLD), or the like.

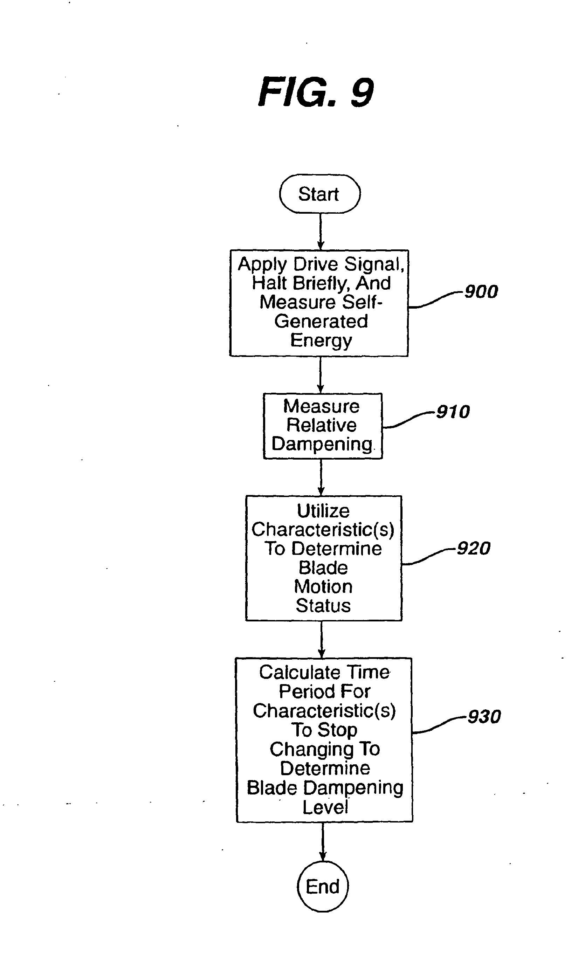

FIG. 9 is a flow chart illustrating an embodiment of the invention. A drive signal is applied to the transducer, briefly halted and piezo self-generated energy is measured, as indicated in step **900.** The relative dampening of the blade based on the energy, voltage, current and/or impedance of a blade which has been driven to operational levels (i.e., levels associated with cutting and cauterizing tissue) is measured, as indicated in step **910.** Here, the relative level of dampening is measured by performing sequential time measurements of the characteristic(s), namely impedance, voltage, current, capacitance of the hand piece/blade. In this case, the console first determines a valid frequency with which to measure the characteristic(s) which are not corrupted by unwanted resonances. Next, the blade is driven at resonance and the drive signal is abruptly removed. The characteristics are measured at least once over a period of time, such as three hundred milliseconds. The measured characteristics are influenced by the yet-vibrating blade, and this effect becomes less pronounced as the motion of the blade subsides. The sequential characteristic measurements are used to indicate relative blade motion status, as indicated in step **920.** The level of dampening is determined by calculating the time period required for the characteristic(s) to stop changing or the speed at which characteristic(s) changes, as indicated in step **930.**

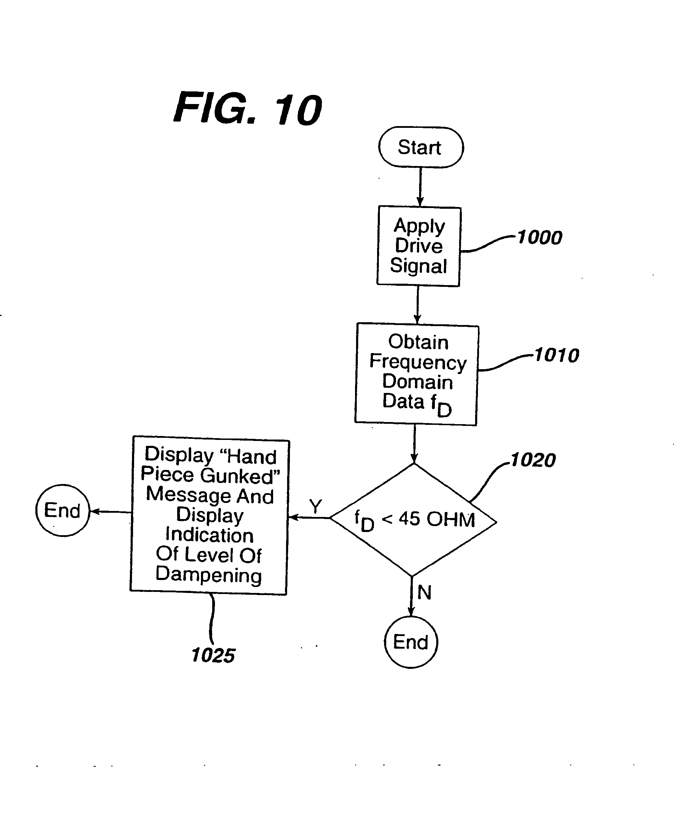

FIG. 10 is a flow chart illustrating a further embodiment of the invention. Here, the relative level of blade dampening is determined using frequency domain measurements. An unusually low system Q is an indication of the presence of debris in the sheath or the occurrence of high blade loading. Accordingly, the hand piece/blade system is driven at a given level, as indicated in step **1000.** Frequency domain measurements are performed to obtain frequency domain data f_{D}, as indicated in step **1010.** If f_{D} is less than 45 ohms (step **1020**), then a "Blade is Gunked" message is displayed on the LCD 12, as indicated in step **1025.** The frequency domain measurements f_{D} are also used to provide an indication of the presence of debris in the sheath or the occurrence of high blade loading. The debris dampens the blade vibrations, and also reduces the Q of the hand piece/blade system. Thus, debris is detected by measuring the extent of blade dampening or the reduction of the Q of the hand piece/blade. This effect is pronounced while the blade is held "in the air," since the variable causes of dampening are mostly related to debris. In particular, contact with tissue will load or dampen the blade. If the blade is held in air so it does not touch the tissue, only the gunk will load the blade. This measurement can be obtained when initiated/directed by the user and/or automatically when the impedance of the hand piece/blade is distinctly high, thus indicating that the blade is not in contact with tissue.

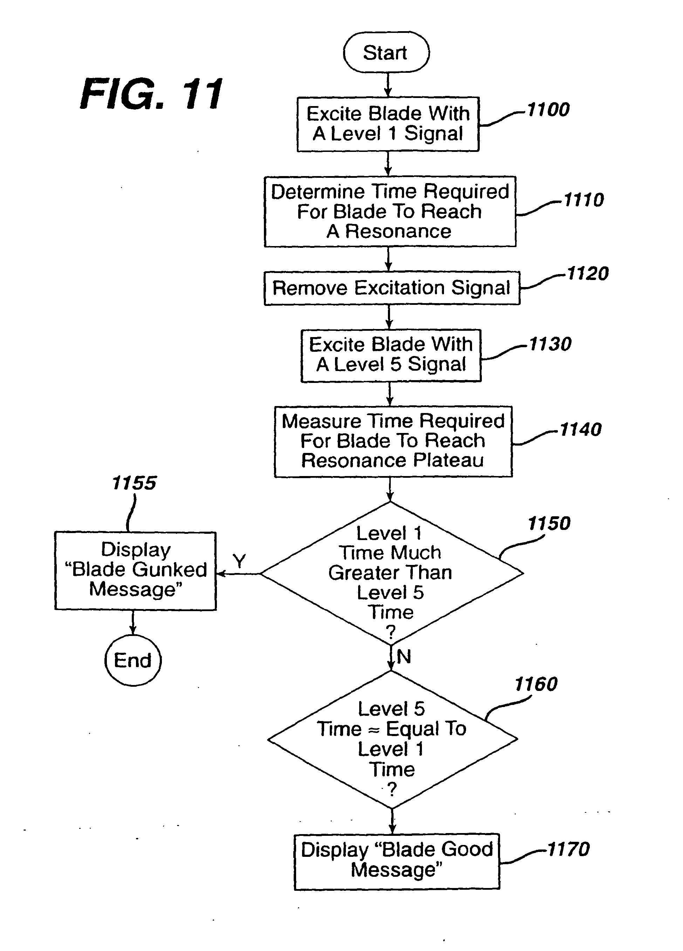

FIG. 11 is a flow chart illustrating an additional embodiment of the invention. In this case, the relative level of dampening is measured by sequentially driving the hand piece/blade with increasingly larger or decreasingly smaller amounts of energy. A more dampened blade requires a greater amount of energy to begin resonating. Here, the relative level of energy required to enter/exit resonance is used to indicate the amount of hand piece/blade dampening. Accordingly, under control of the program stored in the DSP or microprocessor 60 shown in FIGS. 8(a) and 8(b), the method of the invention is implemented by exciting the blade with a level 1 signal, such as 282 mA peak or 200 mA RMS, as indicated in step **1100.**

The time required for the blade to reach a resonance plateau is determined, as indicated in step **1110.** The excitation signal to the blade is then removed, as indicated in step **1120.** A level 5 excitation signal, such as 564 mA peak or 425 mA RMS, is applied to the blade, as indicated in step **1130.** The time required for the blade to reach a resonance plateau is determined, as indicated in step **1140.** A comparison of the time to reach each plateau when driven by a level 1 signal and a level 5 signal is performed, as indicated in step **1150.** If the time for the blade to reach a resonance plateau when it is excited with the level 1 signal is much greater than the time for the blade to reach a resonance plateau when it is excited with the level 5 signal, then gunk exists on the blade, and a "Blade Gunked" message is displayed on the LCD 12, as indicated in step **1155.** On the other hand, if the time for the blade to reach a resonance plateau when it is excited with the level 5 signal is approximately equal to the time for the blade to reach a resonance plateau when it is excited with the level 1 signal (step **1160**), then the blade okay, and a "Blade is Good" message is displayed on the LCD 12, as indicated in step **1170.**

The relative level of dampening is measured while initially driving the blade with a low level of energy which is then rapidly increased. Next, the period of time for the displacement to reach a target value is measured. The displacement measurements are obtained by performing relative comparisons between electrical measurements of the magnitude of the lowest impedance obtained, the maximum phase between the current and the voltage, the resonance frequency of the blade, and/or an evaluation of the non-linearity and/or continuousness of the measured data.

Using a method related to, but separate from the invention, the state of a blade (i.e., whether the blade is cracked, gunked or good) during use in an operation room can be determined quickly, easily and accurately. The method(s) makes this determination independent of the type of hand piece/blade, the temperature of the hand piece/blade or the age of PZT, etc. The method also expedites the testing of unknow blades since less characteristic(s) data points are required to make conclusions due to the acquisition of blade-specific information. The method informs a surgeon or nurse whether to discard a broken hand piece/blade, while also providing an opportunity to clean a gunked blade.

Although the invention has been described and illustrated in detail, it is to be clearly understood that the same is by way of illustration and example, and is not to be taken by way of limitation. The scope of the present invention is to be limited only by the terms of the appended claims.

## Claims

1. A method for determining a damping level of a hand piece/blade in an ultrasonic system, comprising the steps of:
applying a drive signal to a transducer of a hand piece/blade;
halting the drive signal briefly;
measuring piezoelectric energy generated by motion of the hand piece/blade (900),
measuring a relative dampening of the hand piece/blade by performing sequential time measurements of the hand piece/blade characteristics wherein the characteristics of the hand piece/blade is at least one of impedance, voltage, current and capacitance (910);
determine blade motion status using blade characteristics (920), and
calculating a damping level of the hand piece/blade using one of a time period required for the blade characteristics to stop changing and a speed at which the blade characteristics change (930).

2. The method of claim 1, wherein said performing step comprises the steps of:
determining a valid frequency with which to measure the characteristics which are not corrupted by unwanted resonances;
driving the hand piece/blade at resonance and abruptly removing the drive signal; and
measuring the characteristics at least once over a period of time.

3. The method of claim 2, wherein the period of time is three hundred milliseconds.

4. A method for determining a relative dampening level of a blade in an ultrasonic system, comprising the steps of:
driving a hand piece/blade using an ultrasonic generator (1000);
performing frequency domain measurements of the hand piece/blade to obtain frequency domain data (1010);
comparing the frequency domain data to a predetermined threshold; and
if the frequency domain data is less than the predetermined level (1020), displaying a message on a liquid crystal display of the generator (1025).

5. The method of claim 4, wherein the step of displaying the message comprises displaying a "Hand Piece Gunked" message and displaying a level of hand piece/blade damping on the liquid crystal display.

6. The method of claim 4, wherein the predetermined level is approximately 45 ohms.

7. The method of claim 4, wherein the measurements are obtained when at least one which is initiated by a user and automatically when an impedance of the hand piece/blade is distinctly low.

8. A method for determining relative level of dampening of a hand piece/blade in an ultrasonic system, comprising the steps of:
driving the hand piece/blade at a first signal level using an ultrasonic generator (1100);
determining a first time for the hand piece/blade to reach a resonance plateau (1110);
removing the drive signal from the hand piece/blade (1120);
driving the hand piece/blade at a second signal level using the ultrasonic generator (1130);
determining a second time for the hand piece/blade to reach the resonance plateau (1140);
comparing the first time to the second time;
if the first time is substantially greater than the second time (1150), displaying a first message on a liquid crystal display of the generator (1155); and
if the first time is approximately equal to the second time (1160); displaying a second message on a liquid crystal display of the generator (1170).

9. The method of claim 8, wherein the first message is a "Blade Gunked" message.

10. The method of claim 8, wherein the second message is a "Blade is Good" message.

11. The method of claim 8, wherein the first signal level is approximately one of 282 mA peak and 200 mA RMS.

12. The method of claim 8, wherein the second signal level is approximately one of 564 mA peak and 425 mA RMS.

## Patentansprüche

1. Verfahren zum Bestimmen eines Dämpfungspegels eines Handstückes/einer Klinge in einem mit Ultraschall arbeitenden System, das die Schritte aufweist:
Anlegen eines Treibersignals an einen Transducer eines Handstückes/einer Klinge;
kurzes Unterbrechen des Treibersignals;
Messen piezoelektrischer Energie, die durch die Bewegung des Handstückes/der Klinge (900) erzeugt worden ist;
Messen einer relativen Dämpfung des Handstückes/der Klinge durch Ausführen aufeinander folgender zeitlicher Messungen von Kenngrößen des Handstückes/der Klinge, wobei die Kenngrößen des Handstückes/der Klinge wenigstens eine aus Impedanz, Spannung, Strom und Kapazität (910) ist;
Bestimmen des Bewegungszustandes der Klinge, wobei Kenngrößen (920) der Klinge verwendet werden; und
Berechnen eines Dämpfungspegels des Handstückes/der Klinge, wobei eines aus einem Zeitintervall, das erforderlich ist, damit die Kenngröße der Klinge aufhört, sich zu ändern, und einer Geschwindigkeit, mit der sich die Kenngröße der Klinge ändert (930), verwendet wird.

2. Verfahren nach Anspruch 1, bei dem der Ausführungsschritt die Schritte aufweist:
Bestimmen einer gültigen Frequenz, mit der die Kenngrößen zu messen sind, die nicht durch unerwünschte Resonanzen unbrauchbar gemacht werden;
Treiben des Handstückes/der Klinge in die Resonanz und plötzliches Abnehmen des Treibersignals; und
Messen der Kenngröße wenigstens einmal über eine Zeitdauer.

3. Verfahren nach Anspruch 2, bei dem die Zeitdauer dreihundert Millisekunden beträgt.

4. Verfahren zum Bestimmen eines relativen Dämpfungspegels einer Klinge in einem mit Ultraschall arbeitenden System, das die Schritte aufweist:
Treiben eines Handstückes einer Klinge, wobei ein Ultraschallgenerator verwendet wird (1000);
Durchführen von Frequenzdomänenmessungen bei dem Handstück/der Klinge, um Frequenzdomänendaten zu erhalten (1010);
Vergleichen der Frequenzdomänendaten mit einem vorbestimmten Schwellenwert; und
falls die Frequenzdomänendaten kleiner sind als der vorbestimmte Pegel (1020), Anzeigen einer Nachricht auf einer Flüssigkristallanzeige des Generators (1025).

5. Verfahren nach Anspruch 4, bei dem der Schritt des Anzeigens der Nachricht das Anzeigen einer Nachricht "Handstück verklebt" und das Anzeigen eines Pegels der Dämpfung von Handstück/Klinge auf der Flüssigkristallanzeige aufweist.

6. Verfahren nach Anspruch 4, bei dem der vorbestimmte Pegel ungefähr 45 Ohm beträgt.

7. Verfahren nach Anspruch 4, bei dem die Messungen erhalten werden, wenn sie von einem Benutzer eingeleitet werden, und/oder automatisch, wenn eine Impedanz des Handstückes/der Klinge merklich niedrig ist.

8. Verfahren zum Bestimmen eines relativen Pegels der Dämpfung bei einem Handstück/einer Klinge in einem mit Ultraschall arbeitenden System, das die Schritte aufweist:
Treiben des Handstückes/der Klinge bei einem ersten Signalpegel, wobei ein Ultraschallgenerator verwendet wird (1100);
Bestimmen eines ersten Zeitraumes für das Handstück/die Klinge, in dem ein Resonanzplateau erreicht wird (1110);
Abnehmen des Treibersignals von dem Handstückes/der Klinge (1120);
Treiben des Handstückes/der Klinge bei einem zweiten Signalpegel, wobei der Ultraschallgenerator verwendet wird (1130);
Bestimmen eines zweiten Zeitraumes für das Handstück/die Klinge, in dem das Resonanzplateau erreicht wird (1140);
Vergleichen des ersten Zeitraumes mit dem zweiten Zeitraum;
wenn der erste Zeitraum wesentlich größer ist als der zweite Zeitraum (1150), Anzeigen einer ersten Nachricht auf einer Flüssigkristallanzeige des Generators (1155); und
wenn der erste Zeitraum ungefähr gleich dem zweiten Zeitraum ist (1150), Anzeigen einer zweiten Nachricht auf einer Flüssigkristallanzeige des Generators (1170).

9. Verfahren nach Anspruch 8, bei dem die erste Nachricht eine Nachricht "Klinge verklebt" ist.

10. Verfahren nach Anspruch 8, bei dem die zweite Nachricht eine Nachricht "Klinge ist gut" ist.

11. Verfahren nach Anspruch 8, bei dem der erste Signalpegel ungefähr einer mit 282 mA Spitzenwert und 200 mA RMS ist.

12. Verfahren nach Anspruch 8, bei dem der zweite Signalpegel ungefähr einer mit 564 mA Spitzenwert und 425 mA RMS ist.

## Revendications

1. Méthode de détermination d'un niveau d'amortissement d'une pièce manuelle/lame dans un système à ultrasons, comprenant les étapes consistant à _{:}
➢ appliquer un signal de commande à un transducteur d'une pièce manuelle/lame ;
➢ arrêter le signal de commande brièvement ;
➢ mesurer l'énergie piézoélectrique générée par le mouvement de la pièce manuelle/lame (900) ;
➢ mesurer un amortissement relatif de la pièce manuelle/lame en effectuant des mesures temporelles séquentielles des caractéristiques de la pièce manuelle/lame, dans lequel les caractéristiques de la pièce manuelle/lame sont au moins un élément parmi l'impédance, la tension, le courant et la capacité électrique (910) ;
➢ déterminer l'état de mouvement de la lame en utilisant des caractéristiques de la lame (920) ; et
➢ calculer un niveau d'amortissement de la pièce manuelle/lame en utilisant un des laps de temps requis pour que les caractéristiques de la lame arrêtent de changer et une vitesse à laquelle les caractéristiques de la lame changent (930).

2. Méthode selon la revendication 1, dans laquelle ladite étape de réalisation comprend les étapes consistant à _{:}
➢ déterminer une fréquence valable avec laquelle il convient de mesurer les caractéristiques qui ne sont pas corrompues par des résonances indésirables ;
➢ commander la pièce manuelle/lame à une résonance donnée et arrêter soudainement le signal de commande ; et mesurer les caractéristiques au moins une fois sur un laps de temps donné.

3. Méthode selon la revendication 2, dans laquelle le laps de temps est de trois cents millisecondes.

4. Méthode de détermination d'un niveau d'amortissement relatif d'une lame dans un système à ultrasons comprenant les étapes consistant à _{:}
➢ commander une pièce manuelle/lame en utilisant un générateur à ultrasons (1000) ;
➢ effectuer des mesures de domaine de fréquence de la pièce manuelle/lame pour obtenir des données de domaine de fréquence (1010) ;
➢ comparer les données de domaine de fréquence à un seul prédéterminé ; et
➢ si les données de domaine de fréquence sont inférieures à un seuil prédéterminé (1020), afficher un message sur un écran à cristaux liquides du générateur (1025).

5. Méthode selon la revendication 4, dans laquelle l'étape consistant à afficher le message comprend l'affichage d'un message "Pièce manuelle encrassée" et l'affichage d'un niveau d'amortissement de pièce manuelle/lame sur l'écran à cristaux liquides.

6. Méthode selon la revendication 4, dans laquelle le niveau prédéterminé est d'approximativement 45 ohms.

7. Méthode selon la revendication 4, dans laquelle les mesures sont obtenues quand au moins l'une d'elles est lancée par un utilisateur et automatiquement quand une impédance de la pièce manuelle/lame est distinctement basse.

8. Méthode de détermination d'un niveau relatif d'amortissement d'une pièce manuelle/lame dans un système à ultrasons, comprenant les étapes consistant à :
➢ commander la pièce manuelle/lame avec un premier niveau de signal en utilisant un générateur à ultrasons (1100) ;
➢ déterminer un premier moment pour que la pièce manuelle/lame atteigne un plateau de résonance (1110) ;
➢ supprimer le signal de commande de la pièce manuelle/lame (1120) ;
➢ commander la pièce manuelle/lame avec un second niveau de signal en utilisant le générateur à ultrasons (1130) ;
➢ déterminer un second moment pour que la pièce manuelle/la lame atteigne le plateau de résonance (1140) ;
➢ comparer le premier moment au second moment ;
➢ si le premier moment est sensiblement supérieur au second moment (1150), afficher un premier message sur un écran à cristaux liquides du générateur (1155) et
➢ si le premier moment est approximativement égal au second moment (1160), afficher un second message sur un écran à cristaux liquides du générateur (1170).

9. Méthode selon la revendication 8, dans laquelle le premier message est un message "Lame encrassée".

10. Méthode selon la revendication 8, dans laquelle le second message est un message "La lame est bonne".

11. Méthode selon la revendication 8, dans laquelle le premier niveau de signal est d'approximativement 282 mA de pic ou 200 mA de RMS.

12. Méthode selon la revendication 8, dans laquelle le second niveau de signal est d'approximativement 564 mA de pic ou 425 mA de RMS.
